# EUROPEAN PATENT SPECIFICATION

(11) **EP 4 291 298 B1**
(45) Date of publication and mention of the grant of the patent: **20.11.2024**
(21) Application number: 22705030.9
(22) Date of filing: 02.02.2022
(51) Int. Cl.: A61N 1/372, A61B 5/00

(54) **5G IMPLANT WITH PREDETECTION AND/OR WITH A 5G MODEM**
5G-IMPLANTAT MIT VORDETEKTION UND/ODER MIT EINEM 5G-MODEM
IMPLANT 5G AVEC PRÉDÉTECTION ET/OU AVEC UN MODEM 5G

(30) Priority: 09.02.2021 DE 202021100625 U
(43) Date of publication of application: 20.12.2023
(73) Proprietor: BIOTRONIK SE & Co. KG, 12359 Berlin (DE)
(72) Inventor: DOERR, Thomas, 12437 Berlin (DE)
(74) Representative: Biotronik Corporate Services SE
(86) International application number: PCT/EP2022/052386
(87) International publication number: WO 2022/171496

(56) References cited:
- US-A1- 2013 165 819
- US-A1- 2015 119 846
- US-B2- 10 500 401

## Description

The present invention relates to a medical device, in particular an active medical implant.

If medical devices and in particular active electronic implants are influenced in real time by means of 5G network connectivity, a certain amount of additional power is required for this network connectivity, which cannot be guaranteed/accepted on a permanent basis for some product systems, such that this requires appropriate power management for the network connection.

Furthermore, current active medical devices with limited power supply capabilities are regularly unable to connect directly to a cellular network or equivalent. However, as 5G network technology is introduced, this need will increase significantly as control and diagnostic concepts for active medical devices experience a shift of algorithms from the medical device to cloud-based computing systems. Document US-A-2013/165819 discloses the most relevant prior art.

Based on the foregoing, the invention is directed to providing a medical device that is improved with respect to at least one of the above problem areas.

This task is solved by a medical device having the features of claim 1. Further preferred embodiments of the invention are described below. The invention is defined in claims 1 and 14. Any embodiment, which is in contradiction to the subject-matter of claims 1 or 14, is not part of the invention.

According to claim 1, corresponding to a first aspect of the invention, a medical device is disclosed, in particular with limited power supply, such as an implantable active implant for implantation in human or animal tissue, wherein the medical device comprises at least one function for evaluating signals or comprises at least one function for delivering therapy, and in particular at least one of these functions requires timely regulation or control or evaluation for correct function, and wherein the medical device comprises an interface to a network, preferably to a 5G network or to a comparable or higher level network, wherein the interface is directly or indirectly connected to the network, e.g. a 5G network on demand and via the network communication, e.g. 5G network communication direct or indirect influence can be exerted on at least one of the functions of the medical device, and wherein the medical device comprises a predetection unit that activates a connection to the network only whenever an immediate need for real-time assessment or influence has been detected.

In other words, the invention describes the integration of active implants and implant systems or comparable medical devices into 5G-based networks and subsequent network generations, wherein at least one of the implant or device functions is indirectly or directly influenced, controlled and/or evaluated via the 5G network in real time or near real time, wherein a pre-criterion must be met prior to activation of the 5G real-time influence.

The invention thus solves the problem that active electronic medical devices with limited energy budget, in particular active implants comprising real-time influencing by 5G network connectivity, use the available energy budget for 5G network communication efficiently, i.e., only when immediately needed.

According to the invention, the predetection unit is influenced with respect to its sensitivity by a remaining energy supply of the medical device.

Furthermore, according to an embodiment of the invention, it is provided that the network, in particular the 5G network, has at least a bandwidth of greater than 100 Mbit/sec. Furthermore, according to an embodiment of the invention, it is provided that the network, in particular the 5G network, has at least a typical latency of less than 10 ms.

Furthermore, according to an embodiment of the invention, it is provided that the medical device is an active electronic implant for permanent implantation.

Further, according to an embodiment of the invention, it is provided that the medical device is an active electronic implant for temporary implantation.

Further, according to one embodiment of the invention, it is provided that the medical device is a battery-powered electronic device for temporary or permanent attachment to the body.

Further, according to one embodiment of the invention, it is provided that the medical device is one of the following medical devices:
- a cardiac pacemaker;
- a wireless pacemaker;
- an implantable defibrillator, transvenous or non-transvenous;
- a neurostimulator, of any design;
- a cardiac rhythm monitor;
- an implantable sensor for physiological parameters;
- an implantable communication system, e.g., as a relay station for communication with one or more other implants;
- an implant for monitoring medical prostheses;
- an implant for monitoring patient activity or compliance; and
- an implant for monitoring the patient's medication intake.

Furthermore, according to one embodiment of the invention, said interface of the medical device is formed by a first communication interface used for communication between the medical device and a further communication device, and by the further communication device connected to the network, in particular the 5G network, via a second communication interface.

Furthermore, according to one embodiment of the invention, it is provided that the interface of the medical device is directly connected or connectable to the network, in particular the 5G network.

According to a further embodiment of the invention, it is provided that the medical device or implant comprises a detection unit for signaling interruptions of a connection to the network, e.g., the 5G network, which activates a basic supply unit that ensures a basic function of the medical device.

Finally, according to another embodiment of the invention, it is provided that the medical device or implant comprises a connection quality analysis unit that can predict a probable interruption of a connection to the network and thus activates or pre-initializes the basic supply unit in advance.

A second aspect of the present disclosure, which is not part of the invention, relates to a medical device, in particular with limited power supply, such as an implantable active implant for implantation in human or animal tissue, wherein the medical device is provided with a modem for direct communication with a network, in particular a 5G network or a comparable or higher level network.

In other words, this second aspect of the disclosure describes the integration of active implants and implant systems or comparable medical devices into preferably 5G-based networks and subsequent network generations, wherein the implant can dial directly into the 5G network according to the invention, i.e., has a 5G modem and preferably has corresponding functions for energy management.

This second aspect of the disclosure solves in particular the problem of directly connecting active medical devices with limited power supply capabilities to 5G-based networks (or higher).

Furthermore, a third aspect of the disclosure, which is not part of the invention, relates to a medical device, in particular with limited power supply, such as for example an implantable active implant for implantation in human or animal tissue, wherein the medical device comprises at least one function for evaluating signals or at least one function for delivering therapy, and at least one of these functions requires timely regulation or control or evaluation for correct function, and wherein the medical device comprises an interface to a network, in particular to a 5G network or to a comparable or higher level network, wherein the interface is adapted to be able to communicate directly with the network, e.g. the 5G network, on demand and via the network communication, e.g. 5G network communication, at least one of the aforementioned functions of the medical device can be directly or indirectly influenced, and wherein the medical device has an interface designed for the network communication, e.g. 5G network communication exclusively or non-exclusively allocated power management unit, and, optionally, a power supply unit adapted to power the network communication, e.g., 5G network communication, in an energetically proportionate manner to the overall function of the medical device.

The second and third aspects of the disclosure may further be supplemented or further illustrated by the following embodiments.

Accordingly, one embodiment provides a power management unit that manages allowable power budgets or limits for operating network communications (e.g., 5G network communications) and enables operation only when the corresponding budget is still available or the limit is not exceeded.

These budgets and limits are preferably differentiated depending on the importance of the current network communication, in particular 5G network communication, i.e., there are function- or situation-dependent budgets and/or limits.

According to a further embodiment, the power management unit manages / accomplishes a recharging of a rechargeable power source of the medical device to provide network communication, e.g., 5G network communication, and controls the enabling of the network communication depending on the state of charge of the rechargeable power source.

According to a further embodiment, it is provided that the power management unit allows network communication (e.g., 5G network communication) always (only) when an energy-intensive function of the medical device is imminent or already taking place, e.g., the charging process of an ICD.

Furthermore, according to one embodiment, it is provided that the network, in particular the 5G network, has at least a bandwidth of greater than 100 Mbit/sec.

Furthermore, according to an embodiment, it is provided that the network, in particular the 5G network, has at least a typical latency of less than 10ms.

Furthermore, according to an embodiment, it is provided that the medical device is an active electronic implant for permanent implantation.

Further according to an embodiment, it is provided that the medical device is an active electronic implant for temporary implantation.

Further, according to one embodiment, it is provided that the medical device is a battery-powered electronic device for temporary or permanent attachment to the body.

Further, according to one embodiment, the medical device is one of the following medical devices: an active implant, a catheter, a pump, an imaging system, an active VI system, a pacemaker, a wireless pacemaker, a transvenous or non-transvenous implantable defibrillator, a neurostimulator of any type, a cardiac rhythm monitor, an implantable sensor for physiological parameters, an implantable communication system (e.g., as a relay station for communication with one or more other implants), or an implant for monitoring (e.g., medical prostheses, patient activity or compliance, patient medication). e.g., as a relay station for communication with one or more further implants), or an implant for monitoring (e.g., medical prostheses, patient activity or compliance, patient medication).

Furthermore, according to one embodiment of the invention, said interface of the medical device is formed by a first communication interface, which is used for communication between the medical device and a further communication device, and by the further communication device, which is connected or connectable to the network, in particular the 5G network, via a second communication interface.

Furthermore, according to one embodiment of the invention, it is provided that the interface of the medical device is directly connected or connectable to the network, in particular the 5G network.

Finally, according to one embodiment, it is provided that the medical device comprises a detection unit for signaling interruptions of a connection to the network, in particular the 5G network, which is adapted to activate a basic supply unit of the medical device, which ensures a basic function of the medical device. Furthermore, according to one embodiment, it is provided that the medical device comprises a connection quality analysis unit that can predict a probable interruption of a connection to the network, e.g. the 5G network, and thus activates or pre-initializes the basic supply unit in advance.

For a more complete understanding of the present invention and advantages thereof, reference is now made to the following description taken in conjunction with the accompanying drawings. The invention is explained in more detail below using exemplary embodiments, which are specified in the schematic figures of the drawings, in which:
- Fig. 1: shows a schematic representation of a 5G network controlled medical device in the form of an electronic implant;
- Fig. 2: shows a schematic representation of an embodiment of a first aspect of the invention;
- Fig. 3: shows a schematic representation of an embodiment of a second aspect of the invention; and
- Fig. 4: shows a schematic representation of an embodiment of a third aspect of the invention.

5G network technology is characterized by a significantly higher data rate and, more importantly, significantly lower latency (~1 ms). Likewise, the spatial availability of this network technology is expected to improve significantly compared to the current network coverage. In addition to established applications for remote data transmission, 5G technology will support real-time applications for the first time, such as autonomous driving.

Here, the invention particularly exploits the fact of a very short latency of this network technology in order to be able to realize real-time control of medical devices and in particular electronic implants with significantly more complex algorithms and principles.

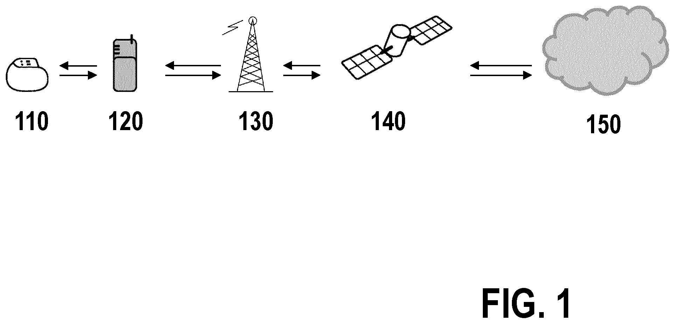

Fig. 1 illustrates a medical device 110 in the form of a 5G network-controlled electronic implant, hereinafter referred to as implant 110. This first transmits to a patient's own relay station 120 sensor data that the implant 110 has recorded. The relay station 120 may, for example, take the form of a cell phone or a smart phone. This initial transmission path is required for energy reasons, as the power supply and antenna arrangement (body attenuation) of this implant 110 are not designed for direct communication with a 5G network.

The sensor data is then transmitted to a 5G network via base station 130 and a 5G satellite 140 to a cloud-based real-time evaluation system 150, where it is evaluated and control signals derived from it are transmitted back to the implant 110 in real time via the aforementioned communication system, so that an implant function, such as therapy delivery or therapy adjustment, can be performed directly here. The difference between this and automated remote programming of an implant is that the influence on the control function occurs in near real-time, and thus essential implant algorithms for implant control can be relocated to the cloud-based evaluation system 150.

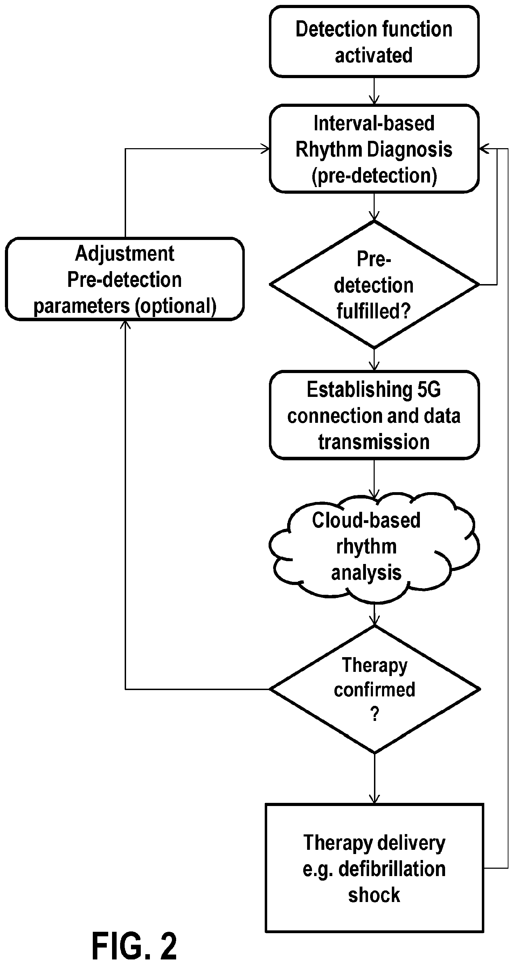

In Fig. 2, an embodiment of the first aspect of the invention is shown schematically, in the form of a non-transvenous defibrillator. In such a system, the challenge is to have to make a defibrillation therapy decision on a signal very similar to the surface ECGs. These signals are influenced by many factors, such as externally coupled disturbances, muscle potentials, changes in patient position, motion artifacts, etc., so that current systems have limited sensitivity and specificity with respect to defibrillation therapy. If such a defibrillator is realized according to the invention, the ECG signal(s) will be sent to the cloud-based evaluation system before a therapy decision is made if there is a sufficient need for therapy, said signals will be evaluated with considerably more complex algorithms, and within a very short time the implant's therapy decision will be confirmed or revoked.

The flow chart for the therapy decision is shown in Fig. 2. In this context, according to the invention, the establishment of the 5G connection is carried out by a predetection unit, which activates the 5G network connection only when there is an immediate need for real-time evaluation or influence, here exemplified by a cloud-based rhythm analysis. The invention thus enables completely new, more complex possibilities for controlling and evaluating medical devices, in particular active implants, addressing in particular the problem of the energetically costly 5G network connection when the energy supply of the medical device is limited.

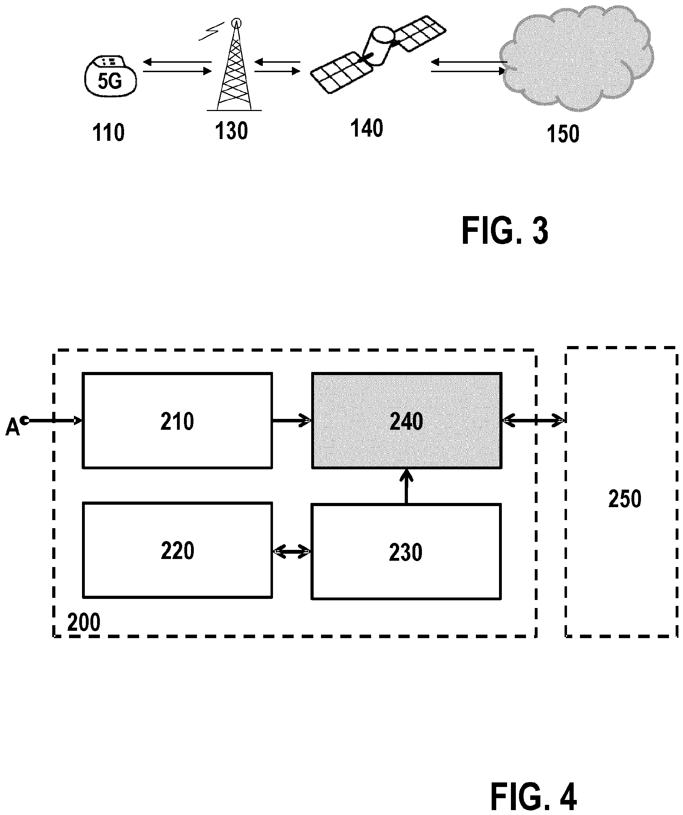

In Fig. 3, an embodiment of the second aspect of the invention is illustrated by means of a 5G network-controlled electronic implant 110. According to the invention, this transmits sensor data recorded by the implant 110 directly by means of a 5G modem to a 5G network via base station 130 and a 5G satellite 140 and subsequently to a cloud-based real-time evaluation system 150.

The control signals evaluated there and derived therefrom in real time are transmitted back to the implant 110 via the aforementioned communication system, so that one or more implant functions can be adjusted here in real time, such as therapy delivery or therapy adjustment. The difference between this and automated remote programming of an implant is that the influence on the control function is near real-time, and thus key implant algorithms for implant control can be moved to the cloud-based evaluation system 150.

Finally, Fig. 4 shows a block diagram of an embodiment of a medical device 200 or medical product according to the second or third aspect of the invention. This has a sensor and/or therapy device 210 for recording body-related or procedure-related sensor signals, typically connected to a device A directly or indirectly contacting/touching or influencing the body tissue. Further, the device or medical device 200 has a power source 220 and an (optional) power management unit 230 and a communication unit 240 for direct communication with a 5G network 250. The communication unit 240 is implemented as a 5G modem, which has a power consumption owed to the network communication and cannot be activated without limitation in case of limited capacities of the power source 220.

In this block diagram, for example, an implantable defibrillator (ICD) is shown. Here, the therapy device 210 corresponds to the detection and therapy control unit of the ICD. The power source 220 is the primary battery of the ICD. Here, the power management unit 230 is configured to establish a network connection to a 5G network only whenever a charging operation of the shock capacitors of the ICD therapy device 210 is imminent.

Such a charging process has a significantly larger energy requirement than a 5G network communication for confirming the therapy decision, so that here the energy use for the "online" confirmation of the therapy decision is classified as appropriate by the power management unit 230 and the communication is enabled.

Again, the invention enables entirely new, more complex ways of controlling and evaluating medical devices, particularly active implants, without the need for an additional relay device to communicate on the 5G network.

### Reference Signs

- 110: medical device
- 120: relay station
- 130: base station
- 140: 5G satellite
- 150: cloud-based real-time evaluation system
- 200: medical device
- 210: therapy device
- 220: power source
- 230: power management unit
- 240: communication unit
- 250: 5G network
- A: device

## Claims

1. Medical device (110; 200), in particular implantable active implant for implantation in human or animal tissue, comprising:
- a unit adapted to perform at least one of the following functions: evaluation of signals and therapy delivery;
- an interface to a network (120, 130, 140, 150), in particular a 5G network, wherein the interface can communicate directly or indirectly with the network as required and the at least one function can be influenced directly or indirectly via the network communication; and
- a predetection unit that activates a connection to the network only whenever an immediate need for real-time assessment and/or influence has been detected by the predetection unit,
**characterized in that**
the predetection unit is influenced with respect to its sensitivity by a remaining energy supply of the medical device (110).

2. Medical device of claim 1, wherein the network, in the particular 5G network, has at least a bandwidth of greater than 100 Mbit/sec and/or has at least a typical latency of less than 10 ms.

3. Medical device of any one of the preceding claims, wherein the medical device (110) is an active electronic implant for permanent or temporary implantation.

4. Medical device of any one of the preceding claims, wherein the medical device (110) is a battery-powered electronic device for permanent or temporary attachment to the body.

5. Medical device of any one of the preceding claims, wherein the medical device (110) is one of a cardiac pacemaker, a wireless pacemaker, an implantable defibrillator, in particular transvenous or non-transvenous, a neurostimulator, a cardiac rhythm monitor, an implantable sensor for physiological parameters, an implantable communication system, in particular as a relay station (120) for communication with one or more other implants, an implant for monitoring medical prostheses, an implant for monitoring patient activity or compliance and an implant for monitoring the patient's medication intake.

6. Medical device of any one of the preceding claims, wherein the interface comprises a first communication interface used for communication between the medical device (110) and a further communication device, and a further communication device connected to the network, in particular the 5G network, via a second communication interface.

7. Medical device of any one of the preceding claims, wherein the interface is directly connected or connectable to the network, in particular the 5G network.

8. Medical device of any one of the preceding claims, wherein the medical device (110) comprises a detection unit for signaling interruptions of a connection to the network, in particular the 5G network, which activates a basic supply unit that provides a basic function of the medical device (110).

9. Medical device of claim 8, wherein the medical device (110) comprises a connection quality analysis unit configured to predict a probable interruption of a connection to the network and configured to activate the basic supply unit in advance.

10. Medical device of any one of the preceding claims, wherein the medical device (110) is equipped with a modem for direct communication with a network, in particular the 5G network or a comparable or higher-level network.

11. Medical device of any one of the preceding claims, wherein the medical device (110) comprises a power supply unit adapted to power the network communication in an energetic proportion to the overall function of the medical device (110).

12. Medical device of any one of the preceding claims, wherein the medical device (200) comprises a power management unit (230) that manages allowable power budgets or limits for operating network communications and enables operation only when the corresponding budget is still available or the limit is not exceeded.

13. Medical device of claim 12, wherein the power management unit (230) is adapted to recharge a rechargeable power source (220) of the medical device (200) to provide network communication and is adapted to control activation of network communication depending on a state of charge of the rechargeable power source (220).

14. Computer-implemented method for operating a medical device (110; 200), in particular an implantable active implant for implantation in human or animal tissue, comprising the steps of:
performing (S1) an evaluation of signals by means of a unit;
communicating (S2) directly or indirectly with a network as required and the evaluation of signals can be influenced directly or indirectly via the network communication by means of an interface to the network (120, 130, 140, 150), in particular a 5G network;
and
activating (S3) a connection to the network only whenever an immediate need for real-time assessment and/or influence has been detected by means of a predetection unit;
**characterized in that**
the predetection unit is influenced with respect to its sensitivity by a remaining energy supply of the medical device (110).

## Patentansprüche

1. Medizinisches Gerät (110; 200), insbesondere implantierbares aktives Implantat zur Implantation in menschliches oder tierisches Gewebe, umfassend:
- eine Einheit, die eingerichtet ist, um mindestens eine der folgenden Funktionen auszuführen: Auswertung von Signalen und Verabreichung einer Therapie;
- eine Schnittstelle zu einem Netzwerk (120, 130, 140, 150), insbesondere einem 5G-Netzwerk, wobei die Schnittstelle je nach Bedarf direkt oder indirekt mit dem Netzwerk kommunizieren kann und die mindestens eine Funktion direkt oder indirekt über die Netzwerkkommunikation beeinflusst werden kann; und
- eine Vorerkennungseinheit, die eine Verbindung zum Netzwerk nur dann aktiviert, wenn von der Vorerkennungseinheit ein unmittelbarer Bedarf an einer Echtzeitbewertung und/oder -beeinflussung festgestellt wurde,
**dadurch gekennzeichnet, dass** die Vorerkennungseinheit hinsichtlich ihrer Empfindlichkeit von einem verbleibenden Energievorrat des medizinischen Geräts (110) beeinflusst wird.

2. Medizinisches Gerät nach Anspruch 1, wobei das Netzwerk, insbesondere das 5G-Netzwerk, mindestens eine Bandbreite von mehr als 100 Mbit/s und/oder mindestens eine typische Latenz von weniger als 10 ms aufweist.

3. Medizinisches Gerät nach einem der vorhergehenden Ansprüche, wobei das medizinische Gerät (110) ein aktives elektronisches Implantat zur dauerhaften oder vorübergehenden Implantation ist.

4. Medizinisches Gerät nach einem der vorhergehenden Ansprüche, wobei das medizinische Gerät (110) ein batteriebetriebenes elektronisches Gerät zur dauerhaften oder vorübergehenden Befestigung am Körper ist.

5. Medizinisches Gerät nach einem der vorhergehenden Ansprüche, wobei das medizinische Gerät (110) eines der folgenden ist: ein Herzschrittmacher, ein drahtloser Herzschrittmacher, ein implantierbarer Defibrillator, insbesondere transvenös oder nicht-transvenös, ein Neurostimulator, ein Herzrhythmusmonitor, ein implantierbarer Sensor für physiologische Parameter, ein implantierbares Kommunikationssystem, insbesondere als Relaisstation (120) zur Kommunikation mit einem oder mehreren anderen Implantaten, ein Implantat zur Überwachung medizinischer Prothesen, ein Implantat zur Überwachung der Patientenaktivität oder Compliance und ein Implantat zur Überwachung der Medikamenteneinnahme des Patienten.

6. Medizinisches Gerät nach einem der vorhergehenden Ansprüche, wobei die Schnittstelle eine erste Kommunikationsschnittstelle umfasst, die zur Kommunikation zwischen dem medizinischen Gerät (110) und einem weiteren Kommunikationsgerät verwendet wird, und ein weiteres Kommunikationsgerät, das über eine zweite Kommunikationsschnittstelle mit dem Netzwerk, insbesondere dem 5G-Netzwerk, verbunden ist.

7. Medizinisches Gerät nach einem der vorhergehenden Ansprüche, wobei die Schnittstelle direkt mit dem Netzwerk, insbesondere dem 5G-Netzwerk, verbunden oder verbindbar ist.

8. Medizinisches Gerät nach einem der vorhergehenden Ansprüche, wobei das medizinische Gerät (110) eine Erkennungseinheit zur Signalisierung von Unterbrechungen einer Verbindung mit dem Netzwerk, insbesondere dem 5G-Netzwerk, aufweist, die eine Grundversorgungseinheit aktiviert, die eine Grundfunktion des medizinischen Geräts (110) bereitstellt.

9. Medizinisches Gerät nach Anspruch 8, wobei das medizinische Gerät (110) eine Einheit zur Analyse der Verbindungsqualität umfasst, die so konfiguriert ist, dass sie eine wahrscheinliche Unterbrechung einer Verbindung mit dem Netzwerk vorhersagt und so konfiguriert ist, dass sie die Grundversorgungseinheit im Voraus aktiviert.

10. Medizinisches Gerät nach einem der vorhergehenden Ansprüche, wobei das medizinische Gerät (110) mit einem Modem zur direkten Kommunikation mit einem Netzwerk, insbesondere dem 5G-Netzwerk oder einem vergleichbaren oder höherwertigen Netzwerk, ausgestattet ist.

11. Medizinisches Gerät nach einem der vorhergehenden Ansprüche, wobei das medizinische Gerät (110) eine Energieversorgungseinheit umfasst, die eingerichtet ist, um die Netzwerkkommunikation in einem energetischen Verhältnis zur Gesamtfunktion des medizinischen Geräts (110) mit Energie zu versorgen.

12. Medizinisches Gerät nach einem der vorhergehenden Ansprüche, wobei das medizinische Gerät (200) eine Energieverwaltungseinheit (230) umfasst, die zulässige Energiebudgets oder -grenzen für den Betrieb der Netzwerkkommunikation verwaltet und den Betrieb nur dann ermöglicht, wenn das entsprechende Budget noch verfügbar oder die Grenze noch nicht überschritten ist.

13. Medizinisches Gerät nach Anspruch 12, wobei die Energieverwaltungseinheit (230) eingerichtet ist, eine wiederaufladbare Energiequelle (220) des medizinischen Geräts (200) aufzuladen, um Netzwerkkommunikation bereitzustellen, und eingerichtet ist, die Aktivierung der Netzwerkkommunikation in Abhängigkeit von einem Ladezustand der wiederaufladbaren Energiequelle (220) zu steuern.

14. Computer-implementiertes Verfahren zum Betreiben eines medizinischen Gerätes (110; 200), insbesondere eines implantierbaren aktiven Implantats zur Implantation in menschliches oder tierisches Gewebe, mit den Schritten:
Durchführen (S1) einer Auswertung von Signalen mittels eines Gerätes;
Kommunizieren (S2), je nach Bedarf direkt oder indirekt, mit einem Netzwerk, und die Auswertung der Signale kann direkt oder indirekt über die Netzwerkkommunikation mittels einer Schnittstelle zu dem Netzwerk (120, 130, 140, 150), insbesondere einem 5G-Netzwerk, beeinflusst werden; und
Aktivieren (S3) einer Verbindung zu dem Netzwerk nur dann, wenn mittels einer Vorerkennungseinheit ein unmittelbarer Bedarf für Echtzeitbewertung und/oder - beeinflussung festgestellt wurde;
**dadurch gekennzeichnet, dass** die Vorerkennungseinheit hinsichtlich ihrer Empfindlichkeit von einem verbleibenden Energievorrat des medizinischen Geräts (110) beeinflusst wird.

## Revendications

1. Dispositif médical (110; 200), en particulier un implant actif implantable pour implantation dans un tissu humain ou animal, comprenant :
- une unité adaptée pour réaliser au moins une des fonctions suivantes : évaluation des signaux et administration d'un traitement ;
- une interface vers un réseau (120, 130, 140, 150), en particulier un réseau 5G, dans lequel l'interface peut communiquer directement ou indirectement avec le réseau le cas échéant et l'au moins une fonction peut être influencée directement ou indirectement par le biais de la communication réseau ; et
- une unité de prédétection qui active une connexion au réseau uniquement à chaque fois qu'un besoin immédiat d'évaluation et/ou d'influence en temps réel a été détecté par l'unité de prédétection,
**caractérisé en ce que**
l'unité de prédétection est influencée par rapport à sa sensibilité par une réserve d'énergie restante du dispositif médical (110).

2. Dispositif médical sur la revendication 1, dans lequel le réseau, en particulier le réseau 5G, a au moins une bande passante supérieure à 100 Mbit/s et/ou a au moins une latence typique inférieure à 10 ms.

3. Dispositif médical selon l'une quelconque des revendications précédentes, dans lequel le dispositif médical (110) est un implant électronique actif pour implantation permanente ou temporaire.

4. Dispositif médical selon l'une quelconque des revendications précédentes, dans lequel le dispositif médical (110) est un dispositif électronique alimenté par pile pour fixation permanente ou temporaire au corps.

5. Dispositif médical selon l'une quelconque des revendications précédentes, dans lequel le dispositif médical (110) est un parmi un stimulateur cardiaque, un stimulateur sans câbles, un défibrillateur implantable, en particulier transveineux ou non transveineux, un neurostimulateur, un moniteur de rythme cardiaque, un capteur implantable de paramètres physiologiques, un système de communication implantable, en particulier sous forme d'une station relais (120) pour communication avec un ou plusieurs autres implants, un implant pour surveiller les prothèses médicales, un implant pour surveiller l'activité ou l'observance du patient et un implant pour surveiller la prise de médicaments par le patient.

6. Dispositif médical selon l'une quelconque des revendications précédentes, dans lequel l'interface comprend une première interface de communication utilisée pour la communication entre le dispositif médical (110) et un dispositif de communication supplémentaire, et un dispositif de communication supplémentaire connecté au réseau, en particulier le réseau 5G, par le biais d'une seconde interface de communication.

7. Dispositif médical selon l'une quelconque des revendications précédentes, dans lequel l'interface est directement connectée ou connectable au réseau, en particulier le réseau 5G.

8. Dispositif médical selon l'une quelconque des revendications précédentes, dans lequel le dispositif médical (110) comprend une unité de détection pour signaler les interruptions d'une connexion au réseau, en particulier le réseau 5G, qui active une unité d'alimentation de base qui fournit une fonction de base du dispositif médical (110).

9. Dispositif médical selon la revendication 8, dans lequel le dispositif médical (110) comprend une unité d'analyse de qualité de connexion conçue pour prévoir une interruption probable d'une connexion au réseau et conçue pour activer l'unité d'alimentation de base par avance.

10. Dispositif médical selon l'une quelconque des revendications précédentes, dans lequel le dispositif médical (110) est équipé d'un modem pour communication directe avec un réseau, en particulier le réseau 5G ou un réseau comparable ou de niveau plus élevé.

11. Dispositif médical selon l'une quelconque des revendications précédentes, dans lequel le dispositif médical (110) comprend une unité d'alimentation électrique adaptée pour alimenter la communication réseau dans une proportion énergétique par rapport à la fonction globale du dispositif médical (110).

12. Dispositif médical selon l'une quelconque des revendications précédentes, dans lequel le dispositif médical (200) comprend une unité de gestion d'alimentation (230) qui gère les budgets ou limites d'alimentation acceptables pour faire fonctionner les communications réseau et permet le fonctionnement uniquement lorsque le budget correspondant est encore disponible ou que la limite n'est pas dépassée.

13. Dispositif médical selon la revendication 12, dans lequel l'unité de gestion d'alimentation (230) est adaptée pour recharger une source d'alimentation rechargeable (220) du dispositif médical (200) pour fournir une communication réseau et est adaptée pour commander l'activation de la communication réseau en fonction d'un état de charge de la source d'alimentation rechargeable (220).

14. Procédé mis en oeuvre par ordinateur pour le fonctionnement d'un dispositif médical (110 ; 200), en particulier d'un implant actif implantable pour implantation dans un tissu humain ou animal, comprenant les étapes consistant à :
réaliser (S1) une évaluation des signaux au moyen d'une unité ;
communiquer (S2) directement ou indirectement avec un réseau le cas échéant et l'évaluation des signaux peut être influencée directement ou indirectement par le biais de la communication réseau au moyen d'une interface vers le réseau (120, 130, 140, 150), en particulier un réseau 5G ;
et
activer (S3) une connexion au réseau uniquement à chaque fois qu'un besoin immédiat d'évaluation et/ou d'influence en temps réel a été détecté au moyen d'une unité de prédétection ;
**caractérisé en ce que**
l'unité de prédétection est influencée par rapport à sa sensibilité par une réserve d'énergie restante du dispositif médical (110).
